# EUROPEAN PATENT SPECIFICATION

(11) **EP 0 690 117 B1**
(45) Date of publication and mention of the grant of the patent: **30.01.2002**
(21) Application number: 95303918.7
(22) Date of filing: 07.06.1995
(51) Int. Cl.: C09K 15/20, C07C 15/46, C07C 7/20

(54) **Compositions and methods for inhibiting vinyl aromatic monomer polymerization**
Zusammensetzungen und Verfahren zur Inhibierung der Polymerisierung von vinylaromatischen Monomeren
Compositions et procédés pour inhiber la polymérisation de monomères vinylaromatiques

(30) Priority: 30.06.1994 US 269307; 30.06.1994 US 269308; 24.08.1994 US 295311
(43) Date of publication of application: 03.01.1996
(73) Proprietor: BetzDearborn Europe, Inc., Trevose PA 19053-6783 (US)
(72) Inventor: Arhancet, Graciela Barbieri, Katy, TX 77450 (US)
(74) Representative: W.P. Thompson & Co.

(56) References cited:
- EP-A- 0 240 297
- EP-A- 0 594 341
- DE-B- 1 154 455
- US-A- 4 237 326

## Description

### FIELD OF THE INVENTION

This invention relates to compositions and methods for inhibiting the unwanted polymerization of vinyl aromatic monomers during processing.

### BACKGROUND OF THE INVENTION

Common industrial methods for producing styrene typically include separation and purification processes such as distillation to remove unwanted impurities. Unfortunately, purification processes carried out at elevated temperatures result in an increased rate of undesired polymerization. Distillation is generally carried out under vacuum to minimize loss of monomer. The presence of oxygen, although virtually excluded in styrene distillation, will also promote polymerization of the monomer.

This polymerization results not only in loss of desired monomer end-product, but also in the loss of production efficiency caused by polymer formation and/or agglomeration of polymer on process equipment. Thermal polymerization of styrene monomer results in formation of normal (i.e. linear) polymer. This resulting polystyrene polymer is characterized by its glassy and transparent appearance and its solubility in the styrene monomer and many organic solvents.

### SUMMARY OF THE INVENTION

The present invention provides for methods for inhibiting the polymerization of vinyl aromatic monomers, such as styrene, and compositions comprising synergistic combinations of actives.

According to the present invention there is provided a method for inhibiting the polymerization of vinyl aromatic monomers in an oxygen-free vinyl aromatic monomer processing system comprising adding an effective polymerization inhibiting amount of a dinitrophenol compound, a hydroxylamine compound, and a phenylenediamine compound.

The hydroxylamine compound acts as the polymerization inhibiting compound and the phenylenediamine compound acts as a catalyst in accelerating the reaction between the hydroxylamine compound and free radicals present in the monomer system.

### DESCRIPTION OF THE RELATED ART

The compounds generally used commercially to prevent polymerization of vinyl aromatic monomers are of the dinitrophenolic type. For example, U.S. 4,105,506, Watson, et al., teaches the use of 2,6-dinitro-p-cresol as polymerization inhibitor of vinyl aromatic compounds. U.S. 4,466,905, Butler, et al., teaches that 2,6-dinitro-p-cresol and p-phenylenediamines will inhibit polymerization in the distillation column if oxygen is present. U.S. 4,774,374, Abruscato, et al., teaches compositions and processes for inhibiting the polymerization of a vinyl aromatic compound employing an oxygenated species formed by the reaction of oxygen and a N-aryl-N'-alkyl-p-phenylenediamine. U.S. 4,720,566, Martin, teaches methods and compositions for inhibiting polymerization of acrylonitrile in the quench tower, no oxygen excluded, using a hydroxylamine compound and a phenyl-p-phenylenediamine compound.

Czechoslovakia Patent No. 163,428 teaches a method for stabilizing styrene and divinylbenzene utilizing 2,4-dinitroorthocresol and diethylhydroxylamine. European Patent Application 0 240 297 also teaches the use of this combination to inhibit polymerization of styrene. Both these disclosures treat systems at lower temperatures and higher oxygen contents. The use of diethylhydroxylamine however is problematic in styrene purification processes as it has a boiling point (125°C to 130°C at 760 mm Hg) similar to styrene and will carry over with the styrene during purification processing.

A variety of inhibitor compositions have been employed in styrene and other vinyl aromatic monomers to inhibit undesirable polymerization. Amongst others, agents that have been used include sulfur, p-benzo-quinone, phenylenediamines, tert-butyl pyrocatechol, phenothiazine, hydroxylamines, nitrocompounds, and hindered phenols. However, many of these compounds present disadvantages such as high toxicity, instability and explosion hazard under elevated temperature, or insufficient efficacy under processing conditions (i.e. inhibitor requires oxygen to be effective). The present inventor has discovered a novel method for inhibiting vinyl aromatic monomer polymerization that avoids these problems associated with known inhibitors.

EP-A-0 594 341 discloses a method and composition for inhibiting the polymerisation of vinyl aromatic monomers under distillation conditions. The composition comprises a combination of a phyenylenediamine compound and a hydroxylamine compound.

US-A-4,466,905 discloses a composition for preventing the polymerisation of vinyl aromatic compounds, which composition may include 2,6-dinitro-p-cresol and a phenylenediamine.

EP-A-0 240 297 discloses a method of inhibiting polymerisation in the presence of oxygen of a vinyl aromatic compound by the use of a substituted hydroxylamine having a boiling point higher than that of the vinyl aromatic compound and a dinitrophenol.

### DETAILED DESCRIPTION OF THE INVENTION

As mentioned above the present invention relates to methods for inhibiting the polymerization of vinyl aromatic monomers in an oxygen-free vinyl aromatic monomer processing system comprising adding to the monomers a combination of a dinitrophenol compound, a hydroxylamine compound, and a phenylenediamine compound.

The method of the present invention proves effective at inhibiting the polymerization of vinyl aromatic monomers under monomer processing conditions. These processing conditions include but are not limited to the purification and distillation processes of vinyl aromatic monomers.

The vinyl aromatic monomers that can be treated by the present invention include but are not limited to styrene, bromostyrene, divinylbenzene and α-methylstyrene. The compositions of the present invention are particularly efficacious at inhibiting polymerization of styrene monomer.

The phrase "oxygen-free processing conditions" is meant to define the substantially oxygen free conditions under which vinyl aromatic monomers, particularly styrene, are processed. These conditions, exemplified by distillation and purification processes, generally have less than 2 parts . per million oxygen present and preferably less than 1 part per million oxygen per parts styrene.

The dinitrophenol compounds preferably have the structure 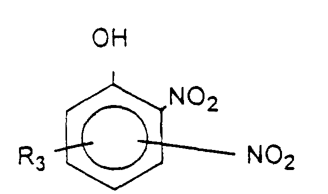 wherein R₃ is hydrogen or C₁ to C₁₂ alkyl.

The hydroxylamine compounds useful in this invention preferably have the formula: 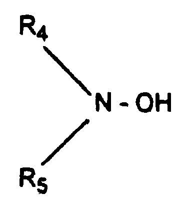 wherein R₄ and R₅ are the same or different and are hydrogen, alkyl, aryl, alkaryl, aralkyl, or hydroxyalkyl groups and preferably have three to about twenty carbon atoms. In one embodiment, R₄ is H and R₅ is C₆ alkyl to C₂₀ alkyl. The preferred hydroxylamine compound is bis-(hydroxypropyl)hydroxylamine (HPHA).

The phenylenediamine compounds useful in this invention preferably have the formula: 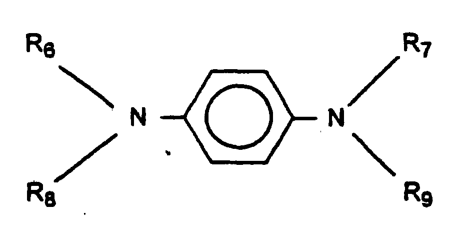 wherein R₆, R₇, R₈ and R₉ are the same or different and are hydrogen, alkyl, aryl, alkaryl or aralkyl groups having one to about twenty carbon atoms. The preferred phenylenediamine compounds are N,N'-di-sec-butyl-p-phenylenediamine and N-phenyl-N'-(1,4-dimethylpentyl)-p-phenylenediamine.

The compositions of the present invention prove effective at inhibiting the polymerization of vinyl aromatic monomers during oxygen-free processing. The inventive compositions provide enhanced activity over each separate component in styrene monomer undergoing distillation and purification processes at elevated temperatures. Styrene is typically processed at temperatures between 95 and 125°C. The compositions of the present invention prove particular efficacy in higher temperature (>110°C) styrene monomer processing systems.

The total amount of dinitrophenol compound, hydroxylamine compound and phenylenediamine compound used in the methods of the present invention is that amount which is sufficient to inhibit polymerization and will vary according to the conditions under which the vinyl aromatic monomer is being processed and exposed to high temperatures. At higher temperature and higher monomer contamination, larger amounts of polymerization inhibiting composition are generally required.

Preferably, the total amount of the composition added to the vinyl aromatic monomer ranges from 1 to about 10,000 parts per million parts monomer. More preferably, the treatment range is from about 5 parts to about 500 parts of the composition per million parts monomer.

The weight ratio of dinitrophenol compound to hydroxylamine to phenylenediamine compound preferably ranges from 1:9:1 to 9:1:9 with a weight ratio of 1:1:1 preferred.

The compositions used in the method of the present invention can be added to the vinyl aromatic monomer by any conventional method, either as individual ingredients or as a combination of ingredients. It is preferred that they are added as a single treatment composition.

The compositions used in the method of the present invention may be added to the vinyl aromatic monomer as either a dispersion or as a solution using suitable liquid carrier or solvent. Any solvent that is compatible with the individual ingredients of the composition and the vinyl aromatic monomer may be employed.

Accordingly, it is possible therefor to produce a more effective vinyl aromatic monomer polymerization inhibition treatment than is obtainable by the use of any one ingredient alone when measured at comparable treatment levels. This enhanced activity allows for the concentration of each of the ingredients to be lowered and the total quantity of polymerization inhibitor required, particularly at higher processing temperatures, may be reduced.

The preferred composition used in the method of the invention employs bis-(hydroxypropyl)hydroxylamine and N,N'-di-sec-butyl-p-phenylenediamine with 4,6-dinitro-o-cresol and 2-sec-butyl-4,6-dinitrophenol, respectively.

This invention will now be further described with reference to a number of specific examples which are to be regarded solely as illustrative and not as restricting the scope of the invention.

### Examples

In order to evaluate the improved polymerization inhibition of the inventive compositions and to demonstrate the enhanced activity of each composition, styrene polymerization testing was performed.

Uninhibited styrene (5 mL) was placed in a test tube and the appropriate amount of treatment was added. The tube was capped with a septum and argon was bubbled through the liquid at 15 mL/min for 3 minutes. Then, the tubes were placed in an oil bath heated to 120°C for 2 hours. The amount of polystyrene formed was determined by methanol precipitation. Results of this testing are summarized in Table I.

**TABLE I**

| | | |
|---|---|---|
| Styrene Polymerization Test | | |
| Uninhibited Styrene | | |
| 120°C | | |

| Treatment | Dose (ppm) | Percent Polymer |
|---|---|---|
| Blank | ------------ | 23.3 |
| DNOC | 300 | 2.15 |
| DNBP | 300 | 1.77 |
| PDA | 300 | 21.06 |
| HPHA | 300 | 17.96 |
| PDA:HPHA:DNOC | 100:100:100 | 1.38 |
| PDA:HPHA:DNOC | 75:150:75 | 1.78 |
| PDA:HPHA:DNOC | 50:150:100 | 1.23 |
| PDA:HPHA:DNBP | 100:100:100 | 1.02 |
| PDA:HPHA:DNBP | 50:150:100 | 0.79 |
| DNOC is 4,6-dinitro-o-cresol | | |
| DNBP is 2-sec-butyl-4,6-dinitrophenol | | |
| PDA is N,N'-di-sec-butyl-p-phenylenediamine | | |
| HPHA is bis-(hydroxypropyl)hydroxylamine | | |

These test results demonstrate the enhanced polymerization inhibition of the three component combination of DNOC/DNBP, HPHA and PDA. Unexpected results were evidenced in a range from 1:1:1 to 1:3:2 at inhibiting styrene polymerization at higher (120°C) styrene processing temperatures.

Uninhibited styrene (100 mL) was placed in a 250-mL three-necked flask fitted with a bubbler, a septa, and a condenser. The appropriate treatment was added and argon was bubbled through the solution at 10 mL/min for 10 minutes. Then, while argon sparging continued, the flask was immersed in an oil bath heated at 120°C. Samples (5.0 mL) were taken every 30 minutes and the amount of polymer formed was determined by methanol precipitation. The results of this testing are presented below

Table II reports the efficacy of the composition used in the method of the present invention at inhibiting styrene polymerization.

**TABLE II**

| | |
|---|---|
| Styrene Polymerization Test | |
| Uninhibited Styrene | |
| 120°C | |
| Treatment: PDA/HPHA/DNBP in a 200:200:100 ppm ratio | |

| Time (hrs.) | % Polymer |
|---|---|
| 1 | 0.07 |
| 2 | 0.31 |
| 3 | 0.49 |
| 4 | 1.10 |
| PDA is N,N'-di-sec-butyl-p-phenylenediamine | |
| HPHA is bis-(hydroxypropyl)hydroxylamine | |
| DNBP is 2-sec-butyl-4,6-dinitrophenol | |

While this invention has been described with particular embodiments thereof, it is apparent that numerous other forms and modifications of this invention will be obvious to those skilled in the art. The appended claims and this invention generally should be construed to cover all such obvious forms and modifications which are within the true spirit and scope of the present invention.

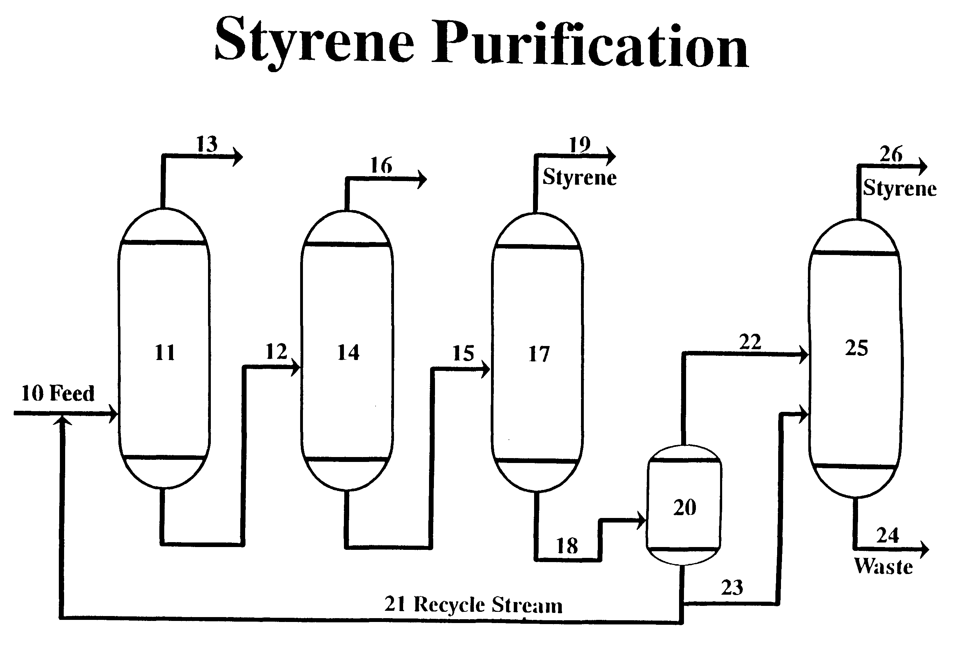

## Claims

1. A method for inhibiting the polymerization of vinyl aromatic monomers in an oxygen-free vinyl aromatic monomer processing system comprising adding an effective polymerization inhibiting amount of a dinitrophenol compound, a hydroxylamine compound, and a phenylenediamine compound.

2. A method as claimed in claim 1, wherein said dinitrophenol compound has the formula 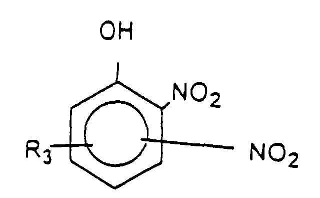 wherein R₃ is hydrogen or C₁ to C₁₂ alkyl.

3. A method as claimed in claim 1, wherein said dinitrophenol compound is selected from the group consisting of 4,6-dinitro-o-cresol, 2,6-dinitro-p-cresol and 2-sec-butyl-4,6-dinitrophenol.

4. A method as claimed in claim 1, 2 or 3, wherein said hydroxylamine compound has the formula: 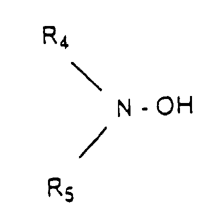 wherein R₄ and R₅ are the same or different and are hydrogen, alkyl, aryl, alkaryl, aralkyl, or hydroxyalkyl groups.

5. A method as claimed in claim 1, 2 or 3, wherein said hydroxylamine compound is bis-hydroxypropylhydroxylamine.

6. A method as claimed in any one of the preceding claims, wherein said phenylenediamine compound has the formula: 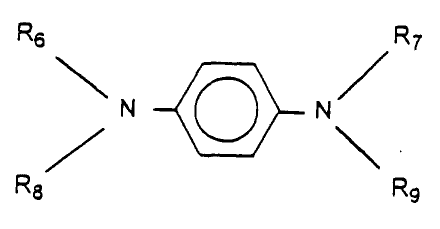 wherein R₆, R₇, R₈ and R₉ are the same or different and are hydrogen, alkyl, aryl, alkaryl or aralkyl groups having one to about twenty carbon atoms.

7. A method as claimed in any one of the preceding claims, wherein said phenylenediamine compound is N, N¹-di-sec-butyl-p-phenylenediamine.

8. A method as claimed in any one of the preceding claims, wherein said dinitrophenol compound, hydroxylamine compound and phenylenediamine compound are added to said vinyl aromatic monomer in an amount ranging from 1 to about 10,000 parts per million parts monomer.

9. A method as claimed in any one of the preceding claims, wherein said vinyl aromatic monomer has a temperature of 110°C or higher.

10. A method as claimed in any one of the preceding claims, wherein said vinyl aromatic monomer is styrene.

## Patentansprüche

1. Verfahren zum Inhibieren der Polymerisation von aromatischen Vinylmonomeren in einem sauerstofffreien aromatischen Vinylmonomer-Verarbeitungssystem, das Folgendes umfasst: Hinzufügen einer wirksamen, die Polymerisation inhibierenden Menge einer Dinitrophenol-Verbindung, einer Hydroxylamin-Verbindung und einer Phenylendiamin-Verbindung.

2. Verfahren nach Anspruch 1, worin genannte Dinitrophenol-Verbindung die folgende Formel aufweist: 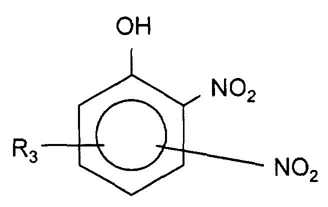 worin R₃ Wasserstoff oder C₁- bis C₁₂-Alkyl ist.

3. Verfahren nach Anspruch 1, worin genannte Dinitrophenol-Verbindung aus der Gruppe ausgewählt wird, die aus 4,6-Dinitro-o-kresol, 2,6-Dinitro-p-kresol und 2-sek.-Butyl-4,6-dinitrophenol besteht.

4. Verfahren nach Anspruch 1, 2 oder 3, worin genannte Hydroxylamin-Verbindung die folgende Formel aufweist: 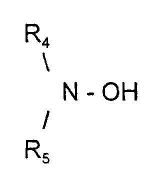 worin R₄ und R₅ gleich oder verschieden sind und Wasserstoff, Alkyl-, Aryl-, Alkaryl-, Aralkyl- oder Hydroxyalkyl-Gruppen sind.

5. Verfahren nach Anspruch 1, 2 oder 3, worin genannte Hydroxylamin-Verbindung Bis-hydroxypropylhydroxylamin ist.

6. Verfahren nach einem der vorangehenden Ansprüche, worin genannte Phenylendiamin-Verbindung die folgende Formel aufweist: 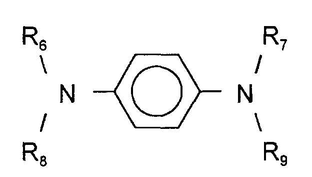 worin R₆, R₇, R₈ und R₉ gleich oder verschieden sind und Wasserstoff, Alkyl-, Aryl-, Alkaryl- oder Aralkyl-Gruppen mit einem Kohlenstoffatom bis ca. zwanzig Kohlenstoffatomen sind.

7. Verfahren nach einem der vorangehenden Ansprüche, worin genannte Phenylendiamin-Verbindung N,N¹-Di-sek.-butyl-p-phenylendiamin ist.

8. Verfahren nach einem der vorangehenden Ansprüche, worin genannte Dinitrophenol-Verbindung, Hydroxylamin-Verbindung und Phenylendiamin-Verbindung genanntem aromatischen Vinylmonomer in einer Menge hinzugefügt werden, die in dem Bereich von 1 Teil bis ca. 10 000 Teile auf 1 Million Teile Monomer liegt.

9. Verfahren nach einem der vorangehenden Ansprüche, worin genanntes aromatische Vinylmonomer eine Temperatur von 110°C oder höher aufweist.

10. Verfahren nach einem der vorangehenden Ansprüche, worin genanntes aromatische Vinylmonomer Styren ist.

## Revendications

1. Méthode pour l'inhibition de la polymérisation de monomères vinyles aromatiques dans un système de transformation de monomères vinyles aromatiques dépourvu d'oxygène, comprenant l'addition d'une quantité, efficace pour l'inhibition de la polymérisation, d'un composé de dinitrophénol, d'un composé d'hydroxylamine et d'un composé de phénylènediamine.

2. Méthode selon la revendication 1, dans laquelle ledit composé de dinitrophénol a la formule 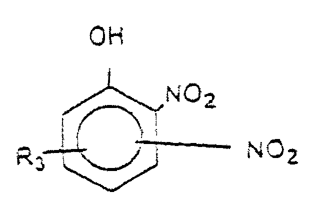 dans laquelle R₃ représente l'hydrogène ou un alkyle en C₁ à C₁₂.

3. Méthode selon la revendication 1, dans laquelle ledit composé de dinitrophénol est choisi parmi le groupe constitué par le 4,6-dinitro-o-crésol, le 2,6-dinitro-p-crésol et le 2-sec-butyl-4,6-dinitrophénol.

4. Méthode selon la revendication 1, 2 ou 3, dans laquelle ledit composé d'hydroxylamine a la formule : 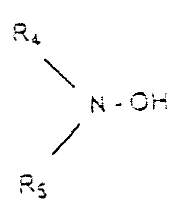 dans laquelle R₄ et R₅ sont identiques ou différents et représentent l'hydrogène ou des groupements alkyle, aryle, alkaryle, aralkyle ou hydroxyalkyle.

5. Méthode selon la revendication 1, 2 ou 3, dans laquelle ledit composé d'hydroxylamine est la bis-hydroxypropylhydroxylamine.

6. Méthode selon l'une quelconque des revendications précédentes, dans laquelle ledit composé de phénylènediamine a la formule : 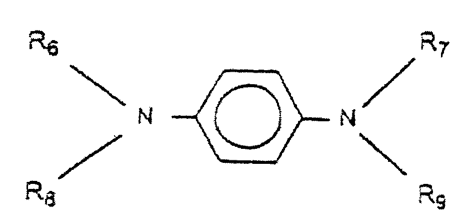 dans laquelle R₆, R₇, R₈ et R₉ sont identiques ou différents et représentent l'hydrogène ou des groupements alkyle, aryle, alkaryle ou aralkyle ayant de un jusqu'à environ vingt atomes de carbone.

7. Méthode selon l'une quelconque des revendications précédentes, dans laquelle ledit composé de phénylènediamine est la N,N¹-di-sec-butyl-p-phénylènediamine.

8. Méthode selon l'une quelconque des revendications précédentes, dans laquelle lesdits composé de dinitrophénol, composé d'hydroxylamine et composé de phénylènediamine sont ajoutés audit monomère vinyle aromatique en une quantité allant de 1 jusqu'à environ 10 000 parties par million de parties de monomère.

9. Méthode selon l'une quelconque des revendications précédentes, dans laquelle ledit monomère vinyle aromatique a une température de 110°C ou plus.

10. Méthode selon l'une quelconque des revendications précédentes, dans laquelle ledit monomère vinyle aromatique est le styrène.
